# EUROPÄISCHE PATENTSCHRIFT

(11) **EP 0 354 204 B1**
(45) Veröffentlichungstag und Bekanntmachung des Hinweises auf die Patenterteilung: **05.04.1995**
(21) Anmeldenummer: 89890202.8
(22) Anmeldetag: 31.07.1989
(51) Int. Cl.: G01N 21/64, G01N 21/80

(54) **Optischer Sensor und Verfahren zu dessen Herstellung**
Optical sensor and process for its production
Senseur optique et procédé de réalisation

(30) Priorität: 04.08.1988 AT 1974/88
(43) Veröffentlichungstag der Anmeldung: 07.02.1990
(73) Patentinhaber: AVL Medical Instruments AG, 8207 Schaffhausen (CH)
(72) Erfinder: Leiner, Marco Jean-Pierre, Dr., A-8010 Graz (AT); Weiss, Leonie, Dr., A-8010 Graz (AT); Wolfbeis, Otto S., Dr., A-8046 Graz (AT)
(74) Vertreter: Krause, Walter, Dr. Dipl.-Ing.

(56) Entgegenhaltungen:
- DE-A- 3 148 830
- DE-A- 3 343 636

## Beschreibung

Die Erfindung betrifft einen optischen Sensor zur Bestimmung zumindest eines Parameters in einer flüssigen oder gasförmigen Probe, mit einer Trägerschicht, welche Trägerpartikel mit einem daran immobilisierten Fluoreszenzindikator aufweist, sowie ein Verfahren zu dessen Herstellung.

Die optische Messung, beispielsweise des pH-Wertes flüssiger Proben, hat in den letzten Jahren bedeutende Fortschritte gemacht, da sie gegenüber elektrochemischen Methoden, z.B. mit Hilfe der Glaselektrode, große Vorteile bietet. So benötigen optische pH-Sensoren keine Referenzzelle und können, in Kombination mit dünnen, faseroptischen Lichtleitern auch für invasive Messungen im Körper eingesetzt werden.

Verschiedene optische pH-Sensoren sind aus der Literatur bekannt. Man unterscheidet prinzipiell zwischen zwei Arten von Sensoren, nämlich solchen, welche zuerst gefertigt und dann am Ende eines Lichtleiters angebracht werden, sogenannte planare Sensoren, oder Fasersensoren erster Art, und solchen, welche direkt durch Modifikation des Lichtleiters hergestellt werden, sogenannte Fasersensoren zweiter Art.

Die Herstellung letztgenannter Sensoren, die nicht Gegenstand dieser Erfindung sind, ist zwar zum Teil eleganter, aber mit technischen Schwierigkeiten verbunden, besonders was eine reproduzierbare Herstellung betrifft. Aus diesem Grund werden für die Massenfertigung eher planare Sensoren bevorzugt.

Ein typischer Sensor der ersten Art ist beispielsweise in Anal.Chem.52, 864 (1980) beschrieben, wo ein pH-sensitives Material, welches am Ende eines Lichtleiters angeordnet ist, zur inversiven Messung von Blut- pH-Werten herangezogen wird. Das sensitive Material wird durch Tränken von Polystyrol-Kügelchen mit einer Indikatorlösung erhalten. Die Befestigung am Ende des Lichtleiters erfolgt durch Füllen eines dünnen, über den Lichtleiter gezogenen Cellophanschlauches mit den Polystyrol-Kügelchen.

Es ist auch möglich, einen Indikator direkt auf eine Membran oder die Oberfläche eines festen Trägers, wie z.B. Glas, aufzubringen. Beispielsweise wird in Sensor and Actuators 9, 73 (1986) von H. Offenbacher et. al. eine Methode zur Immobilisierung von pH-Indikatoren auf Glas, welches am Ende eines Lichtleiters angebracht wird, vorgeschlagen.

Ein weiterer optischer Sensor erster Art ist in DE-A-33 43 636 offenbart. Dort wird ein Fluoreszenzindikator auf Trägerpartikeln z.B. aus Kieselgel immobilisiert. Dann wird auf einem plättchenförmigen, transparenten Träger aus Glas oder Kunststoff Polyurethanlack PU 428, 42%, in Xylol (Fa. VIANOVA KUNSTHARZ) ausgestrichen. Das Immobilisat wird auf den nassen Kunstharzfilm gestreut und vorsichtig gleichmäßig verrieben. Danach wird das Plättchen eine halbe Stunde lang bei 80°C gehärtet und 24 Stunden bei Raumtemperatur getrocknet und anschließend aktiviert. Zur Ausschaltung störender Einflüsse der Probe wird vorgeschlagen, diesen Sensor mit einer Lösung von hydrophilem Polymer, z.B. einer Lösung von Cellulosenitrat in Aceton, zu beschichten, so daß nach Verdunsten des Lösunqsmittels eine hydrophile Polymerschicht zurückbleibt.

Des weiteren ist aus der US-PS 3 904 373 ein pH-Sensor bekannt, welcher Farbstoffindikatoren auf Lichtabsorptionsbasis kovalent an Trägermaterialien gebunden enthält. Die Bindung der Fluoreszenzindikatoren an die Trägermaterialien erfolgt mit funktionellen Triethoxysilanen. Als Trägermaterialien fungieren zusammengesinterte Glaspartikeln, geätztes Glas und CPG-Pulver (controlled porous glass). Nachteiligerweise entstehen beim Sintern von Glaspartikeln frittenartige, wenig transparente Körper mit kleiner spezifischer Oberfläche, welche zudem auf eine Änderung des zu messenden pH-Wertes mit einer zu langen Ansprechzeit reagieren. CPG eignet sich eben falls nur bedingt, da beim Zusammensintern von CPG-Pulver dessen mikroporöse Struktur verlorengeht.

Weitere Nachteile der zur Zeit bekannten optischen Sensoren bestehen darin, daß ihre Herstellung arbeitsaufwendig und daher für Massenproduktionen nur bedingt geeignet ist. Zudem sind die Fluoreszenzindikatoren großteils in eine Umgebung eingebettet, welche vom wäßrigen Probenmaterial nur langsam penetriert wird.

Aufgabe der Erfindung ist es, einen optischen Sensor und ein Verfahren zu dessen Herstellung vorzuschlagen, welcher auf einfache Weise auch in großen Stückzahlen herstellbar ist, auf die sich ändernden Parameter der Probe mit einer kurzen Ansprechzeit reagiert und eine große effektive Oberfläche aufweist, sowie daß der Fluoreszenzindikator in quasiwäßriger Lösung, statt an hydrophobe Materialien gebunden, vorliegt.

Diese Aufgabe wird erfindungsgemäß durch eine Vorrichtung gemäß Anspruch 1 gelöst.

Erfindungsgemäß ist weiter ein Herstellungs verfahren gemäß Anspruch 14 vorgesehen.

Die einzelnen Trägerpartikel werden somit nicht von einem Kleber penetriert sondern durch Druckanwendung in eine Haftschicht eingedrückt und dort lediglich mechanisch fixiert. Der am Trägerpartikel immobilisierte Fluoreszenzindikator ist über die Hydrogelschicht praktisch in allen Bereichen für die zu messenden Substanzen aus der Probe frei zugänglich.

Eine besonders vorteilhafte Ausgestaltung der Erfindung sieht vor, daß die Trägerpartikel aus einzelnen Fasern bestehen, welche mit einem geringen Teil ihrer Oberfläche in der auf der Polymerfolie haftenden Schicht fixiert sind und mit dem größten Teil ihrer Oberfläche in die Hydrogelschicht eintauchen. Gerade bei fasrigem Trägermaterial kann erreicht werden, daß nur ein geringer Teil der Oberfläche in der Haftschicht fixiert wird und der größte Teil der Fasern in die Hydrogelschicht reicht und diese auch gut mechanisch an die Haftschicht bindet.

Es ist erfindungsgemäß auch möglich, daß die Trägerpartikel aus Silicagelkügelchen mit irreversibel gebundenem Fluoreszenzindikator, aus mikroporösen Glaskügelchen oder aus Polyacrylamid bestehen. Dazu eignen sich beispielsweise CPG-Kügelchen oder Polyamidpartikel.

Um eine besonders dünne Haftschicht zu erreichen, ist entsprechend einer Weiterbildung der Erfindung vorgesehen, daß die Haftschicht in einem der gewünschten Schichtstärke entsprechenden Verhältnis mit einem Lösungsmittel verdünnt und unter Zugabe eines Quervernetzers auf die Polymerfolie aufgetragen und eintrocknen gelassen wird, daß die Trägerpartikel mit dem darauf immobilisierten Fluoreszenzindikator gleichmäßig aufgestreut und die Haftschicht bis zu jener Temperatur erhitzt wird, bei welcher diese unter Anwendung von leichtem Druck fließt, daß die Trägerpartikel bis zu angrenzenden Polymerfolie in die Haftschicht eingedrückt und diese abgekühlt wird, daß bei Raumtemperatur zugewartet wird, bis eine Quervernetzung der Haftschicht eintritt, sowie daß eine Hydrogelschicht aufgebracht wird. Typische Verfahrensschritte sind dabei folgende:
1. Aufbringen eines thermoplastisch verformbaren Materials samt Lösungsmittel und Quervernetzer auf einer festen transparenten Polymerfolie in einer definierten Schichtstärke, Lösungsmittel eintrocknen lassen;
2. Aufstreuen der Fasern mit dem daran immobilisierten Fluoreszenzindikator (die Fasern haften noch nicht);
3. Erhitzen des thermoplastisch verformbaren Materials bis zu einer Temperatur, bei der das Material unter Anwendung von leichtem Druck fließt;
4. leichtes Eindrücken der Fasern in das Haftmaterial;
5. Abkühlen (Fasern werden vom Haftmaterial mechanisch fixiert);
6. Mechanisches Entfernen nichthaftender Fasern;
7. 48 Stunden Quervernetzen der Haftschicht bei Raumtemperatur, wobei die Haftschicht duroplastisch wird, sich in wäßrigem Medium nicht mehr auflöst und die Fasern nicht freigibt;
8. Aufbringen einer Hydrogelschicht, wobei die Fasern als Anker wirken.

Erfindungsgemäß entsteht dabei ein Sensor, bei welchem der Faserdurchmesser bzw. der Durchmesser der einzelnen Trägerpartikel größer ist als die Dicke der an der Polymerfolie haftenden Schicht.

Erfindungsgemäß kann in einem weiteren Schritt die Hydrogelschicht von einer pigmentierten oder gefärbten Hydrogelschicht abgedeckt werden, um eine optische Isolierung, beispielsweise zur Abschirmung von Umgebungslicht, Streulicht oder Fluoreszenzlicht aus der Probe zu erreichen. Dazu kann beispielsweise erfindungsgemäße Aktivkohle, Eisenoxidpulver oder Titandioxidpulver verwendet werden.

Zur Erzielung eines pH-Sensors ist vorgesehen, daß ein pH-sensitiver Fluoreszenzindikator an den Trägerpartikeln immobilisiert ist. Andererseits kann zur Messung des CO₂-Partialdruckes eine probenseitig anbringbare CO₂-permeable, ionenimpermeable Membran und ein CO₂-sensitiver Fluoreszenzindikator vorgesehen sein. Auf diese Art kann der CO₂-Partialdruck über Änderungen des inneren pH-Wertes gemessen werden. Dabei kann der Sensor erfindungsgemäß mit einer Silicon-Polycarbonat-Folie abgedeckt sein.

Es ist erfindungsgemäß auch möglich, daß zur Messung von Sauerstoff eine probenseitig anbringbare, O₂-permeable, ionenimpermeable Membran und ein O₂-sensitiver Fluoreszenzindikator vorgesehen wird.

Erfindungsgemäß ist auch die Herstellung eines optischen Enzymsensors denkbar, wenn an die Trägerpartikel oder in der Hydrogelschicht ein Enzym aus der Gruppe der Hydrolasen, Oxidasen und Dehydrogenasen immobilisiert wird.

Besondere Herstellungsvorteile ergeben sich erfindungsgemäß dann, wenn die Haftschicht, die Trägerpartikel samt immobilisiertem Fluoreszenzindikator, die Hydrogelschicht und gegebenenfalls die CO₂- oder O₂-permeable, ionenimpermeable Membran großflächig auf eine Polymerfolie aufgebracht und eine Vielzahl gleichartiger Sensoren in einem Arbeitsgang ausgestanzt werden. Da alle verwendeten Materialien gestanzt bzw. geschnitten werden können, ist die Herstellung von Sensoren beliebiger Formen und Größen auf einfache Weise möglich.

Eines besondere Ausgestaltung des Sensors nach der Erfindung sieht vor, daß die Fasern aus mikrokristallinen Cellulosefasern mit kovalent immobilisiertem Fluoreszenzindikator bestehen, wobei erfindungsgemäß die einzelnen Cellulosefasern einen Durchmesser von 5 bis 10 »m, vorzugsweise ca. 8 »m und eine Länge von 50 bis 200 »m, vorzugsweise ca. 80 »m aufweisen.

Die so hergestellten planaren optischen Sensoren, welche sehr einfach am Ende eines Lichtleiters oder Lichtleiterbündels angebracht werden können, sind dadurch ausgezeichnet, daß die Polymerfolie eine Dicke von 20 bis 500 »m, die darauf haftende Schicht eine Dicke von 5 bis 10 »m und die einzelnen Hydrogelschichten eine Dicke von 5 bis 30 »m aufweisen.

Während somit das direkte Aufbringen von Fluoreszenzfarbstoffen auf Cellulose den Nachteil aufweist, daß eine langsamere Kinetik stattfindet, da sich das Proton bei der Einstellung des pH-Gleichgewichtes zwischen Probe und Sensorschicht nur langsam durch die Cellulosemembran bewegt, erzielt man bei Verwendung von Fasern, beispielsweise von Cellulosefasern, eine größere Oberfläche und eine schnellere Einstellzeit, da sich das Proton relativ rasch durch das Hydrogel bewegen kann, bevor es auf den an die Fasern gebundenen Fluoreszenzindikator trifft. Generell beobachtet man bei Fasern eine schnellere Kinetik und geringere Hystereseeffekte als bei Membranen, sowie einen guten Haftverbund durch Quervernetzung und mechanische Verkrallung der Fasern in der Hydrogelschicht, eine gute Langzeitstabilität des Klebeverbundes, und einen konstanten pKₐ-Wert des Fluoreszenzfarbstoffes im fertigen Sensor.

Ein weiterer Vorteil dieses Sensortyps besteht schließlich darin, daß die optische Isolierung, welche zur Unterdrückung von Falschlicht oft erforderlich ist, ein integraler Bestandteil der Sensorschicht ist, während sie bei anderen Sensortypen in einem eigenen Arbeitsgang mechanisch befestigt werden muß.

Im folgenden wird die Erfindung anhand von Zeichnungen näher erläutert. Es zeigen:
Fig. 1 einen pH-Sensor nach der Erfindung,
Fig. 2 einen erfindungsgemäßen CO₂-Sensor und
Fig. 3 ein weiteres Ausführungsbeispiel eines planaren optischen Sensors, jeweils in schematischer Darstellung.

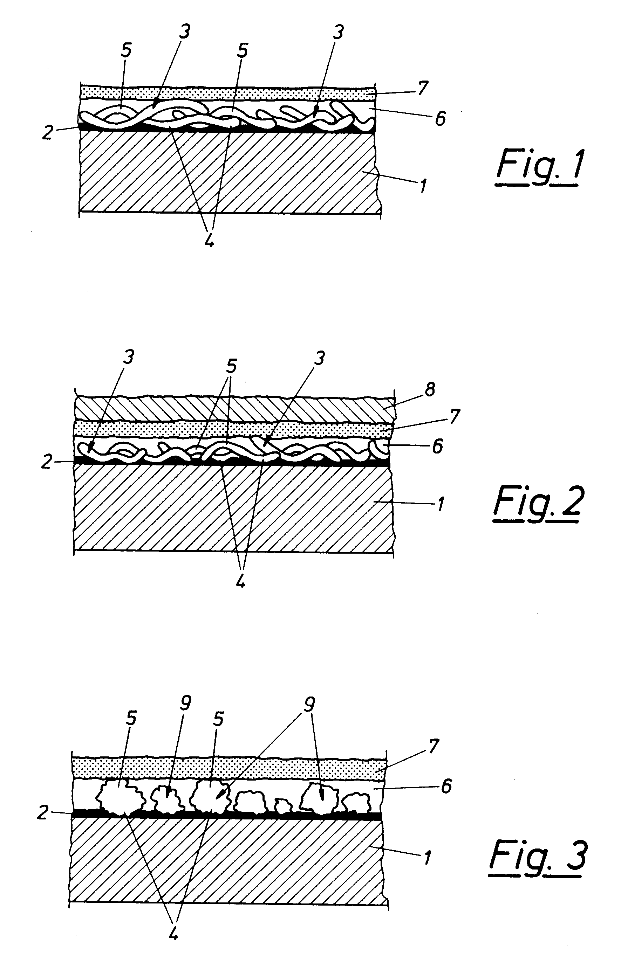

Fig. 1 zeigt einen pH-Sensor, welcher als Trägerschicht eine Polymerfolie 1, beispielsweise eine für die Anregungs- und Emissionsstrahlung durchlässige Mylarfolie, mit einer Dicke von 175 »m aufweist. Darauf befindet sich eine Schicht 2 eines thermoplastisch verformbaren Materials, in welches die einzelnen Fasern 3 mit dem daran immobilisierten Fluoreszenzindikator mit einem Teil 4 ihrer Oberfläche eingedrückt und mechanisch fixiert sind. Der größte Teil 5 der Fasern 3 ragt in eine die Haftschicht 2 bedeckende Hydrogelschicht 6, wodurch der Fluoreszenzindikator in einer quasiwäßrigen Umgebung vorliegt. Als Fluoreszenzindikator kann beispielsweise 1-Hydroxypyren-3,6,8-trisulfonat, Fluorescein, oder ein in 3-Stellung substituiertes Cumarin verwendet werden. Die Fasern 3 dienen gleichzeitig als Immobilisatträger und für eine ausreichende mechanische Verankerung der Hydrogelschicht 6 auf der Haftschicht 2, welche eine Dicke aufweist, die kleiner ist als der Durchmesser der einzelnen Cellulosefasern.

Probenseitig ist eine mit Aktivkohle pigmentierte Hydrogelschicht 7 angeordnet. Zur Anfärbung bzw. Pigmentierung dieser Schicht eignen sich natürlich auch alle anderen in diesem Zusammenhang bekanntgewordenen Substanzen.

Der in Fig. 2 dargestellte CO₂-Sensor, bei welchem gleiche Teile mit gleichen Bezugszeichen versehen sind, unterscheidet sich vom pH-Sensor lediglich dadurch, daß dieser mit einer Phosphat- bzw. Bicarbonatpufferlösung von pH= 8 bis 11 getränkt wird und diese Schicht dann probenseitig mit einer CO₂-permeablen, ionenimpermeablen Membran 8, beispielsweise einer Silicon-Polycarbonat-Copolymerisat-Folie mit einer Dicke von 1 mil abgedeckt wird.

Fig. 3 zeigt schließlich einen optischen Sensor, bei welchem Trägerpartikel 9 aus nichtfasrigem Material Verwendung finden. Die Trägerpartikel können beispielsweise aus Silicagel-Kügelchen mit darin absorbiertem Fluoreszenzindikator, aus mikroporösen Glaskügelchen (CPG) oder aus Polyamid bestehen.

## Patentansprüche

1. Optischer Sensor zur Bestimmung zumindest eines Parameters in einer flüssigen oder gasförmigen Probe, mit einer Trägerschicht, welche Trägerpartikel mit einem daran immobilisierten Fluoreszenzindikator aufweist, wobei die Trägerschicht aus einer für die Anregungs- und Emissionsstrahlung durchlässigen Polymerfolie besteht, wobei die einzelnen Trägerpartikel (3;9) mit dem daran immobilisierten Fluoreszenzindikator lediglich mit einem Teil (4) ihrer Oberfläche in einer auf der Polymerfolie (1) haftenden Schicht (2) fixiert sind, welche als thermoplastisch verformbares Material samt Lösungsmittel aufgebracht wurde, in welcher nach dem Eintrocknen des Lösungsmittels die Trägerpartikel durch thermische Druckverformung mechanisch fixiert wurden und welche nach dem Eindrücken der Trägerpartikel (3;9) duroplastische Eigenschaften annahm, und wobei die einzelnen Trägerpartikel (3;9) mit dem anderen Teil (5) ihrer Oberfläche in eine die haftende Schicht (2) bedeckende, optisch durchlässige Hydrogelschicht (6) eintauchen, welche durch Verkrallung an den Trägerpartikeln (3;9) haftet.

2. Optischer Sensor nach Anspruch 1, **dadurch gekennzeichnet,** daß die Trägerpartikel (3) aus einzelnen Fasern bestehen, welche mit einem geringen Teil (4) ihrer Oberfläche in der auf der Polymerfolie (1) haftenden Schicht (2) fixiert sind und mit dem größten Teil (5) ihrer Oberfläche in die Hydrogelschicht (6) eintauchen.

3. Optischer Sensor nach Anspruch 1, **dadurch gekennzeichnet,** daß die Trägerpartikel (9) aus Silicagelkügelchen mit darin absorbiertem Fluoreszenzindikator, aus mikroporösen Glaskügelchen oder aus Polyamid bestehen.

4. Optischer Sensor nach einem der Ansprüche 1 bis 3, **dadurch** **gekennzeichnet,** daß die optisch durchlässige Hydrogelschicht (6) von einer gefärbten oder mit Pigmenten versehenen Hydrogelschicht (7) bedeckt ist.

5. Optischer Sensor nach einem der Ansprüche 1 bis 4, **dadurch** **gekennzeichnet,** daß ein pH-sensitiver Fluoreszenzindikator an den Trägerpartikeln (3;9) immobilisiert ist.

6. Optischer Sensor nach einem der Ansprüche 1 bis 4, **dadurch** **gekennzeichnet,** daß zur Messung von pCO₂ eine probenseitig anbringbare, CO₂-permeable, ionenimpermeable Membran (8) und ein CO₂-sensitiver Fluoreszenzindikator vorgesehen sind.

7. Optischer Sensor nach einem der Ansprüche 1 bis 4, **dadurch** **gekennzeichnet,** daß zur Messung von Sauerstoff eine probenseitig anbringbare, O₂-permeable, ionenimpermeable Membran und ein O₂-sensitiver Fluoreszenzindikator vorgesehen sind.

8. Optischer Sensor nach einem der Ansprüche 1 bis 4, **dadurch gekennzeichnet,** daß an die Trägerpartikel (3;9) oder in der Hydrogelschicht (6) ein Enzym aus der Gruppe der Hydrolasen, Oxidasen und Dehydrogenasen immobilisiert ist.

9. Optischer Sensor nach einem der Ansprüche 4 bis 8, **dadurch gekennzeichnet,** daß die gefärbte Hydrogelschicht (7) mit Aktivkohle geschwärzt ist.

10. Optischer Sensor nach einem der Ansprüche 1 bis 9, **dadurch gekennzeichnet,** daß der Faserdurchmesser bzw. der Durchmesser der einzelnen Trägerpartikel (9) größer ist als die Dicke der an der Polymerfolie (1) haftenden Schicht (2).

11. Optischer Sensor nach einem der Ansprüche 2 bis 10, **dadurch gekennzeichnet,** daß die Fasern (3) aus mikrokristallinen Cellulosefasern mit kovalent immobilisiertem Fluoreszenzindikator bestehen.

12. Optischer Sensor nach Anspruch 11, **dadurch gekennzeichnet,** daß die einzelnen Cellulosefasern (3) einen Durchmesser von 5 bis 10 »m, vorzugsweise 8 »m und eine Länge von 50 bis 200 »m, vorzugsweise 80 »m aufweisen.

13. Optischer Sensor nach einem der Ansprüche 1 bis 12, **dadurch** **gekennzeichnet,** daß die Polymerfolie (1) eine Dicke von 20 bis 500 »m, die darauf haftende Schicht (2) eine Dicke von 5 bis 10 »m und die einzelnen Hydrogelschichten (6,7) eine Dicke von 5 bis 30 »m aufweisen.

14. Verfahren zur Herstellung eines optischen Sensors, umfassend die Schritte, daß ein thermoplastisch verformbares Material samt Lösungsmittel als dünne Haftschicht auf eine optisch durchlässige Polymerfolie aufgetragen wird, daß nach Eintrocknen des Lösungsmittels die Trägerpartikel mit darauf immobilisiertem Fluoreszenzindikator in gleichmäßiger Verteilung aufgebracht und durch thermische Druckverformung zum Teil in die Haftschicht eingedrückt und von dieser mechanisch fixiert werden, daß die Haftschicht nach dem Eindrücken der Trägerpartikel duroplastische Eigenschaften annimmt, sowie daß eine die Haftschicht bedeckende, die freien Teile der einzelnen Trägerpartikel aufnehmende Hydrogelschicht aufgebracht wird.

15. Verfahren nach Anspruch 14, **dadurch gekennzeichnet,** daß die Haftschicht in einem der gewünschten Schichtstärke entsprechenden Verhältnis mit einem Lösungsmittel verdünnt und unter Zugabe eines Quervernetzers auf die Polymerfolie aufgetragen und eintrocknen gelassen wird, daß die Trägerpartikel mit dem darauf immobilisierten Fluoreszenzindikator gleichmäßig aufgestreut und die Haftschicht bis zu jener Temperatur erhitzt wird, bei welcher diese unter Anwendung von leichtem Druck fließt, daß die Trägerpartikel bis zur angrenzenden Polymerfolie in die Haftschicht eingedrückt und diese abgekühlt wird, daß bei Raumtemperatur zugewartet wird, bis eine Quervernetzung der Haftschicht eintritt, sowie daß eine Hydrogelschicht aufgebracht wird.

16. Verfahren nach einem der Ansprüche 14 bis 15, **dadurch gekennzeichnet,** daß die Hydrogelschicht von einer pigmentierten oder gefärbten Hydrogelschicht abgedeckt wird.

17. Verfahren nach Anspruch 16, **dadurch gekennzeichnet,** daß der Sensor mit einer Silicon-Polycarbonat-Folie abgedeckt wird.

18. Verfahren nach einem der Ansprüche 14 bis 17, **dadurch gekennzeichnet**, daß die Haftschicht, die Trägerpartikel samt immobilisiertem Fluoreszenzindikator, die Hydrogelschicht und gegebenenfalls die CO₂- oder O₂-permeable, ionenimpermeable Membran großflächig auf eine Polymerfolie aufgebracht und eine Vielzahl gleichartiger Sensoren in einem Arbeitsgang ausgestanzt werden.

## Claims

1. An optical sensor for determining one or more parameters in a liquid or gaseous sample, comprising a carrier layer which is provided with carrier particles and a fluorescent indicator immobilized thereon, wherein the carrier layer is configured as a polymer foil transparent to both excitation and emission radiation, and wherein the individual carrier particles (3;9) carrying the fluorescent indicator in immobilized form are attached with only part (4) of their surface to a layer (2) adhering to the polymer foil (1), which layer (2) is formed by applying a thermoplastic material including a solvent, the carrier particles being mechanically attached by the application of heat and pressure after drying of the solvent, and which assumes thermosetting properties after the carrier particles (3;9) have been pressed in, and wherein the individual carrier particles (3;9) extend with the other part of their surface into an optically transparent hydrogel layer (6) covering the adhesive layer (2), in which hydrogel layer (6) the carrier particles (3;9) are anchored mechanically.

2. An optical sensor as in claim 1, characterized in that the carrier particles (3) consist of individual fibers, a small part (4) of whose surface is fixed in the layer (2) adhering to the polymer foil (1), while the larger part (5) of their surface is held in the hydrogel layer (6).

3. An optical sensor as in claim 1, characterized in that the carrier particles (9) are pellets of silica gel in which a fluorescent indicator is absorbed, or beads of microporous glass or polyamide.

4. An optical sensor as in any of claims 1 to 3, characterized in that the optically transparent hydrogel layer (6) is covered by a layer (7) of stained or pigmented hydrogel.

5. An optical sensor as in any of claims 1 to 4, characterized in that a pH-sensitive fluorescent indicator is immobilized in the carrier particles (3;9).

6. An optical sensor as in any of claims 1 to 4, characterized in that for pCO₂ measurement a CO₂-permeable, ion-impermeable membrane (8) to be attached on the sample side, and a CO₂ -sensitive fluorescent indicator are provided.

7. An optical sensor as in any of claims 1 to 4, characterized in that for oxygen measurement an O₂-permeable, ion-impermeable membrane to be attached on the sample side, and an O₂-sensitive fluorescent indicator are provided.

8. An optical sensor as in any of claims 1 to 4, characterized in that an enzyme from the group of hydrolases, oxidases and dehydrogenases is immobilized on the carrier particles (3;9) or in the hydrogel layer (6).

9. An optical sensor as in any of claims 4 to 8, characterized in that the stained hydrogel layer (7) is blackened with activated charcoal.

10. An optical sensor as in any of claims 1 to 9, characterized in that the diameter of the fibers or the diameter of the individual carrier particles (9) is larger than the thickness of the layer (2) adhering to the polymer foil (1).

11. An optical sensor as in any of claims 2 to 10, characterized in that the fibers (3) are microcrystalline cellulose fibers carrying a fluorescent indicator immobilized by covalent bonding.

12. An optical sensor as in claim 11, characterized in that the individual cellulose fibers (3) have a diameter of 5 to 10 micrometers, i.e., preferably 8 micrometers, and a length of 50 to 200 micrometers, i.e., preferably 80 micrometers.

13. An optical sensor as in any of claims 1 to 12, characterized in that the polymer foil (1) has a thickness of 20 to 500 micrometers, while the layer (2) adhering thereto has a thickness of 5 to 10 micrometers, and the individual hydrogel layers (6,7) have a thickness of 5 to 30 micrometers.

14. A method for preparing an optical sensor, comprising the steps that a thin layer of a thermoplastic material including a solvent is coated onto an optically transparent polymer foil, and that after drying of the solvent the carrier particles on which a fluorescent indicator has been immobilized are evenly distributed on and partly pressed into this adhesive layer and mechanically bonded thereto by the application of heat and pressure, and that the adhesive layer assumes themosetting properties after the carrier particles have been pressed in, and further that a hydrogel layer is applied on top of the adhesive layer in order to cover the free upper parts of the individual carrier particles.

15. A method as in claim 14, characterized in that the adhesive layer is diluted with a solvent at a ratio corresponding to the desired thickness of the layer, and that it is coated and left to dry on the polymer foil after the addition of a cross-linking agent, and that the carrier particles carrying the immobilized fluorescent indicator are evenly distributed on the adhesive layer, which is then heated to a temperature at which it starts flowing upon application of a gentle pressure, and that the carrier particles are pressed into the adhesive layer until the adjacent polymer foil is reached, whereupon the adhesive layer is allowed to cool off and cure at room temperature until cross-linkage is complete, and that a hydrogel layer is applied.

16. A method as in claim 14 or 15, characterized in that the hydrogel layer is covered by a pigmented or stained hydrogel layer.

17. A method as in claim 16, characterized in that the sensor is covered with a silicone polycarbonate foil.

18. A method as in any of claims 14 to 17, characterized in that the adhesive layer, the carrier particles including the immobilized fluorescent indicator, the hydrogel layer, and, if provided, the CO₂- or O₂-permeable, ion-impermeable membrane are applied over large areas of a polymer foil, and that a number of identical sensors are simultaneously punched therefrom.

## Revendications

1. Capteur optique pour déterminer au moins un paramètre d'un échantillon liquide ou gazeux, comportant une couche de support munie de particules de support sur lesquelles est immobilisé par un indicateur de fluorescence, dans lequel la couche de support est constituée par une feuille de polymère (1), transparente aux rayonnements d'excitation et d'émission, les différentes particules de support (3, 9) et l'indicateur de fluorescence immobilisé sur ces particules étant uniquement fixés avec une partie (4) de leur surface dans une couche (2) adhérant à la feuille de polymère (1), couche appliquée comme matière thermoplastique déformable avec le solvant et dans laquelle, après séchage du solvant, les particules de support se fixent mécaniquement par déformation thermique par pression et qui après enfoncement des particules de support (3 ; 9), prend des caractéristiques thermodurcissables, et tandis que les différentes particules de support (3, 9) avec l'autre partie (5) de leur surface, pénètrent dans une couche d'hydrogel (6) optiquement transparente, recouvrant la couche adhésive (2), cet hydrogel s'accrochant par effet de griffe aux particules de support (3 ; 9).

2. Capteur optique selon la revendication 1, caractérisé en ce que les particules de support (3) sont constituées de fibres séparées fixées par une faible partie (4) de leur surface dans la couche (2) adhérant sur la feuille de polymère (1) et la plus grande partie (5) de leur surface pénètre dans la couche d'hydrogel (6).

3. Capteur optique selon la revendication 10, caractérisé en ce que les particules de support (9) sont constituées par des petites billes de gel de silice contenant à l'état absorbé un indicateur de fluorescence formé de billes de verre microporeuses ou de polyamide.

4. Capteur optique selon l'une des revendications 1 à 3, caractérisé en ce que la couche d'hydrogel (6) optiquement transparente est recouverte d'une couche d'hydrogel (7) teintée ou chargée de pigments.

5. Capteur optique selon l'une des revendications 1 à 4, caractérisé en ce qu'un indicateur de fluorescence sensible au pH est immobilisé sur les particules de support (3, 9).

6. Capteur optique selon l'une des revendications 1 à 4, caractérisé par une membrane (8) imperméable aux ions, perméable à CO₂, susceptible d'être prévu du côté de l'échantillon pour mesurer la pression partielle pCO₂ et un indicateur de fluorescence sensible à CO₂.

7. Capteur optique selon l'une quelconque des revendications 1 à 4, caractérisé en ce que pour mesurer l'oxygène, on a prévu une membrane imperméable aux ions, perméable à l'oxygène O₂ et qui se fixe du côté de l'échantillon ainsi qu'un indicateur de fluorescence, sensible à l'oxygène O₂.

8. Capteur optique selon l'une des revendications 1 à 4, caractérisé en ce qu'un enzyme du groupe des hydrogénases, des oxydases ou des déhydrogénases est immobilisé sur les particules de support (3, 9) ou dans la couche d'hydrogel (6).

9. Capteur optique selon l'une des revendications 4 à 8, caractérisé en ce que la couche d'hydrogel (7), teintée, est noircie par du charbon actif.

10. Capteur optique selon l'une des revendications 1 à 9, caractérisé en ce que le diamètre des fibres ou le diamètre des différentes particules de support (9) est supérieur à l'épaisseur de la couche (2) adhérant à la feuille de polymère (1).

11. Capteur optique selon l'une des revendications 2 à 10, caractérisé en ce que les fibres (3) sont des fibres de cellulose microcristallines à indicateur de fluorescence covalent, immobilisé.

12. Capteur optique selon la revendication 11, caractérisé en ce que les différentes fibres de cellulose (3) ont un diamètre compris entre 5 et 10 microns, de préférence 8 microns et une longueur de 50 à 200 microns, de préférence 80 microns.

13. Capteur optique selon l'une des revendications 1 à 12, caractérisé en ce que la feuille de polymère (1) a une épaisseur comprise entre 20 et 500 microns et la couche (2) adhérant par-dessus a une épaisseur comprise entre 5 et 10 microns et les différentes couches d'hydrogel (6, 7) ont une épaisseur de 5 à 30 microns.

14. Procédé pour réaliser un capteur optique, comprenant les étapes suivantes : on applique sur une feuille de polymère optiquement transparente, une matière thermoplastique déformable avec le solvant sous la forme d'une couche adhésive mince, après séchage du solvant, les particules de support sur lesquelles a été immobilisé un indicateur de fluorescence, sont appliquées avec une répartition régulière et par déformation thermique par la pression, elles sont comprimées partiellement dans la couche adhésive et fixées mécaniquement à l'aide de celle-ci, et après l'enfoncement des particules de support la couche adhésive prend des caractéristiques thermodurcissables, et une couche d'hydrogel est appliquée qui recouvre la couche adhésive et reçoit les parties libres des différentes particules de support.

15. Procédé selon la revendication 14, caractérisé en ce qu'on applique la couche d'accrochage en la dissolvant avec un solvant suivant un rapport correspondant à l'épaisseur souhaitée de la couche et en ajoutant un agent de réticulation transversale pour appliquer ce mélange sur la feuille de polymère, on laisse sécher, et on disperse régulièrement les particules de support sur lesquelles a été immobilisé l'indicateur de fluorescence et on chauffe la couche adhésive jusqu'à une température pour laquelle cette couche flue sous l'application d'une légère pression, et on enfonce les particules de support dans la couche adhésive jusqu'à la feuille de polymère adjacente et on refroidit, et on attend la température ambiante jusqu'à ce que se produise une réticulation transversale de la couche d'accrochage et on applique une couche d'hydrogel.

16. Procédé selon l'une des revendications 14 à 15, caractérisé en ce que la couche d'hydrogel est recouverte d'une couche d'hydrogel teintée ou contenant des pigments.

17. Procédé selon la revendication 16, caractérisé en ce que le capteur est recouvert d'une feuille de silicium-polycarbonate.

18. Procédé selon l'une des revendications 14 à 17, caractérisé en ce que la couche adhésive, les particules de support ainsi que l'indicateur de fluorescence, immobilisé, la couche d'hydrogel et une membrane imperméable aux ions et perméable le cas échéant à CO₂ ou à O₂, sont appliqués en grande surface sur une feuille de polymère et en ce qu'on estampe au cours d'une phase de travail un grand nombre de capteurs identiques.
